# EUROPÄISCHE PATENTSCHRIFT

(11) **EP 2 462 785 B1**
(45) Veröffentlichungstag und Bekanntmachung des Hinweises auf die Patenterteilung: **29.10.2014**
(21) Anmeldenummer: 10771669.8
(22) Anmeldetag: 31.07.2010
(51) Int. Cl.: H05H 1/24, A61B 18/00, H05H 1/36

(54) **VORRICHTUNG ZUR ERZEUGUNG EINES NICHTTHERMISCHEN ATMOSPHÄRENDRUCK-PLASMAS**
DEVICE FOR GENERATING A NON-THERMAL ATMOSPHERIC PRESSURE PLASMA
DISPOSITIF DE PRODUCTION D'UN PLASMA NON THERMIQUE À PRESSION ATMOSPHÉRIQUE

(30) Priorität: 03.08.2009 DE 102009028190; 21.06.2010 DE 102010030294
(43) Veröffentlichungstag der Anmeldung: 13.06.2012
(73) Patentinhaber: Leibniz-Institut für Plasmaforschung und Technologie e.V., 17489 Greifswald (DE)
(72) Erfinder: KINDEL, Eckhard, 17489 Greifswald (DE); LEMBKE, Norbert, 17489 Greifswald (DE); STIEBER, Manfred, 17489 Greifswald (DE); TITZE, Ruediger, 17489 Greifswald (DE); WELTMANN, Klaus-Dieter, 18609 Binz (DE); HELLWIG, Lutz, 17094 Burg Stargard (DE)
(74) Vertreter: Wehlan, Helmut
(86) Internationale Anmeldenummer: PCT/EP2010/061166
(87) Internationale Veröffentlichungsnummer: WO 2011/015538

(56) Entgegenhaltungen:
- WO-A1-2005/084569
- DE-A1-102006 019 664
- US-A- 3 434 476
- US-B1- 6 325 799

## Beschreibung

Die Erfindung betrifft eine Vorrichtung zur Erzeugung eines kalten, HF-angeregten Plasmas unter Atmosphärendruckbedingungen, umfassend ein im Bereich des austretenden Plasmas als geerdete Elektrode fungierendes Metallgehäuse, in dem ein HF-Generator, eine HF-Resonanzspule mit einem, für die Hochfrequenz geeigneten geschlossenen Ferritkern, ein als Gasdüse fungierender Isolierkörper sowie eine in dem Isolierkörper gehalterte Hochspannungs-Elektrode in der Weise angeordnet sind, dass sie vom Prozessgas um- bzw. durchströmt werden. In einer bevorzugten Ausführungsform umfasst die Vorrichtung eine elektrisch leitende abnehmbare Kappe, die im vorderen Bereich einen Schlitz oder ein Loch aufweist. Die Erfindung kann vorteilhaft für die Plasmabehandlung von Materialien für kosmetische und medizinische Zwecke eingesetzt werden. Die Erfindung ermöglicht es, durch die Integration von Plasmadüse und erforderlicher Ansteuerelektronik in einem miniaturisierten Handgerät oder durch eine Verwendung eines kurzen Hochspannungskabels, eine Einhaltung der Richtlinien der elektromagnetischen Verträglichkeit (EMV) zu gewährleisten und die Verlustleistung zu minimieren und damit einen mobilen Einsatz zu realisieren.

### Stand der Technik

Niedertemperatur-Plasmen werden bereits seit geraumer Zeit zur Behandlung von Oberflächen zum Zweck der Oberflächenaktivierung, des Ätzens, der Polymerisation, zur Schichtabscheidung, zur Reinigung sowie zur Keimreduzierung eingesetzt. Zunächst wurden dafür vorrangig Niederdruckplasmen genutzt, in denen die für die Prozesse erforderlichen reaktiven Spezies durch die Wahl geeigneter Prozessparameter in definiertem Maße erzeugt werden können. Da Niederdruckplasma-Verfahren sowohl aus Kostengründen als auch aus verfahrenstechnischen Gründen für zahlreiche industrielle Prozesse ungeeignet sind, wurden alternative Plasmaverfahren entwickelt, die bei Atmosphärendruck arbeiten, somit wesentlich kostengünstiger sind und sich in entsprechende Fertigungsstrecken einfacher integrieren lassen. Eine Möglichkeit, die für die Anwendbarkeit von Atmosphärendruck-Plasmenverfahren erforderliche Homogenität der Plasmen zu erreichen, besteht darin, durch eine gerichtete Strömung des Arbeitsgases (Prozessgases) einen Plasmastrahl außerhalb des Entladungsraumes zu erzeugen.

Anwendungen von derartigen Strahlplasmen werden in zahlreichen Patentschriften beschrieben. In der Offenlegungsschrift DE 37 33 492 A1 wird beispielsweise eine Vorrichtung zur Erzeugung eines Strahlplasmas beschrieben, bei der ein Gasstrom durch eine Koronaentladungsstrecke zwischen einer stabförmigen Innen- und einer rohrförmigen Außenelektrode durchgeleitet wird. Das in der Patentschrift DE 195 32 412 C2 beschriebene Verfahren zur Plasmabehandlung von Oberflächen basiert auf der Erzeugung eines Plasmastrahls durch einen starken Gasstrom, der durch eine Bogenentladungsstrecke geführt wird. Zur Erzeugung der Atmosphärendruck-Entladungen können dabei verschiedene Arten der Einspeisung der elektrischen Energie genutzt werden. So werden in den Patenschriften US 6,194,036 B1 und US 6,262,523 B1 sowie in der Patentanmeldung US 2002/122896 A1 beispielsweise Anordnungen beschrieben, die auf der HF-Anregung von Atmosphärendruck-Plasmen basieren. Im Bereich der Medizin werden spezielle, HF-angeregte Plasmen bereits seit vielen Jahren zur Koagulation (Argon-Plasma-Koagulation: US 4,781,175 A, US 4,060,088 A, DE 195 13 338 A1) bzw. zur HF-Chirurgie eingesetzt. Es gibt aber auf diesem Gebiet inzwischen auch zahlreiche neuere Entwicklungen, die das Ziel verfolgen, Plasmen dieser Art zum Beispiel auch für die Beschichtung von Implantaten zur Erhöhung ihrer Biokompatibilität sowie zur Steuerung der Zelladhäsion auf Oberflächen (Ohl A., Schröder K.: Plasma assisted surface modification of biointerfaces. In: Hippler R., Kersten H., Schmidt M., Schoenbach K. H. (ed.). Low Temperature Plasmas, Vol. 2. 803-820. Wiley-VCH, Weinheim 2008), zur antimikrobiellen Dekontamination von Oberflächen (R. Brandenburg et al.:Contrib. Plasma Phys. 2007, 47, (1-2), 72-79) sowie zur Behandlung biologischer Zellen und Gewebe (I. E. Kieft et al.: IEEE Transactions on Plasma Science 2005, 33, (2), 771-775) zu nutzen.

Die Erfindung bezieht sich auf eine Vorrichtung der in der Patentschrift des Anmelders "DE 10 2006 019 664 A1" beschriebenen Art. Dort wird eine handliche HF-Plasmadüse beschrieben, bei der aufgrund der speziellen Bauweise auf ein HF-Anpassungsnetzwerk in Form einer separaten Matchbox verzichtet werden kann. Dadurch ist es möglich, eine handliche Bauform der HF-Plasmadüse zu realisieren, die sowohl manuell, als auch durch Roboter geführt werden kann.

Die Verbindung von Plasmadüse und HF-Generator durch ein längeres Kabel führt dazu, dass die Werte der beim Betrieb einer Vorrichtung entsprechend des Standes der Technik auftretenden elektromagnetischen Störstrahlung in der Regel das durch die europäischen Richtlinien zur elektromagnetischen Verträglichkeit (EMV) festgelegte Limit überschreiten. Vorrichtungen dieser Art können nicht ohne einen größeren technischen Aufwand zur Sicherung der Einhaltung dieser Richtlinien eingesetzt werden. Das bedeutet, dass ihr Einsatz auf Anwendungen in Industrieanlagen beschränkt ist, die durch spezielle Maßnahmen hinsichtlich der elektromagnetischen Störstrahlung nach außen abgeschirmt sind. Für den mobilen Einsatz in öffentlichen Bereichen, beispielsweise für medizinische, zahnmedizinische oder kosmetische Zwecke, ist eine Nutzung dieser Vorrichtung nur möglich, wenn sie den EMV-Anforderungen entspricht. Aufgabe der Erfindung ist es, ein Plasmawerkzeug auf der Grundlage eines kalten, HF-angeregten Plasmastrahls zu realisieren, das diesen EMV-Anforderungen genügt und somit für den mobilen Einsatz in Bereichen der Medizin, Zahnmedizin und Kosmetik geeignet ist. Eine weitere Aufgabe besteht darin, in sensibleren Bereichen des Körpers besonders schonend zu arbeiten. bei einer Plasmabehandlung werden insbesondere reaktive Spezies (Radikale) und Strahlung (VUV/UV) erzeugt, die wichtig für den Behandlungseffekt sind. Zum anderen wird das Plasma durch Verlustprozesse aufgeheizt und die Temperatur in der Spitze beträgt etwa 50°C, was unter Plasmabedingungen als "kalt" zu betrachten ist. Bei Anwendungen auf der menschlichen Haut führt jedoch die permanente lokale Behandlung der Haut mit einem Plasma dieser Temperatur zu einer lokalen Verbrennung. Zur Vermeidung derartiger Verbrennungen wird derzeit das Plasma mit einer gewissen Geschwindigkeit über die zu behandelnde Stelle bewegt.

Eine weitere Reizung (Irritation) der Haut, die sich in einem unangenehmen "Kribbeln" äußert, wird durch den elektrischen Strom hervorgerufen, der von der Hochspannungselektrode (3) über das leitende Plasma zur Hautoberfläche fließt. Normalerweise ist dies unbedenklich und mehr oder weniger erträglich. In sensibleren Bereichen des Körpers, wie beispielsweise in der Mundhöhle (im Fall einer Behandlung des Zahnfleisches), ist dies schmerzhaft und nicht mehr zu vertreten

### Aufgabe der Erfindung

Die Aufgabe der Erfindung bestand darin, die Nachteile der im Stand der Technik genannten Lösungen zu beseitigen.

### Lösung der Aufgabe

Die Aufgabe wurde gemäß den Merkmalen der Patentansprüche gelöst.

Erfindungsgemäß wurden alle für den Betrieb des Plasmawerkzeuges erforderlichen Baugruppen, einschließlich HF-Generator, derart miniaturisiert, dass sie in einem miniaturisierten Handgerät integriert werden können. Damit wurde ein Gerät bereitgestellt, das für den mobilen Einsatz in den Bereichen Medizin, Zahnmedizin und Kosmetik geeignet ist. Die erforderliche Gleichspannungsversorgung kann dabei entweder von einem externen Gerät oder intern erfolgen.

Die Hauptursache für eine erhöhte elektromagnetische Störabstrahlung ist die lange Kabelverbindung zwischen der Plasmadüse und einem externen HF-Generator. Der Vorteil der Erfindung besteht vor allem darin, dass durch die Integration von Plasmadüse, Hochspannungsspule und HF-Generator in einem handlichen Plasmawerkzeug diese Kabelverbindung entfällt und damit das Problem der elektromagnetischen Verträglichkeit gelöst wird, so dass die technischen Voraussetzungen für eine Zulassung für den mobilen Einsatz, beispielsweise für medizinische, zahnmedizinische und kosmetische Zwecke, erfüllt ist. Als ein weiterer Vorteil ergibt sich aus der Miniaturisierung eine bessere Handhabbarkeit des Plasmawerkzeuges, das damit vor allem für punktgenaue, lokale Mikroplasmabehandlungen geeignet ist. Darüber hinaus wird durch die Erfindung eine weitgehende Reduzierung der Verlustleistung erreicht. Das hat zur Folge, dass die Erwärmung des Plasmawerkzeuges auf ein Minimum reduziert wird und die im Plasma umgesetzte Leistung, und damit die Länge des Plasmastrahls, allein durch Regelung der dem integrierten HF-Generator extern zugeführten Gleichspannung deutlich variiert werden kann.

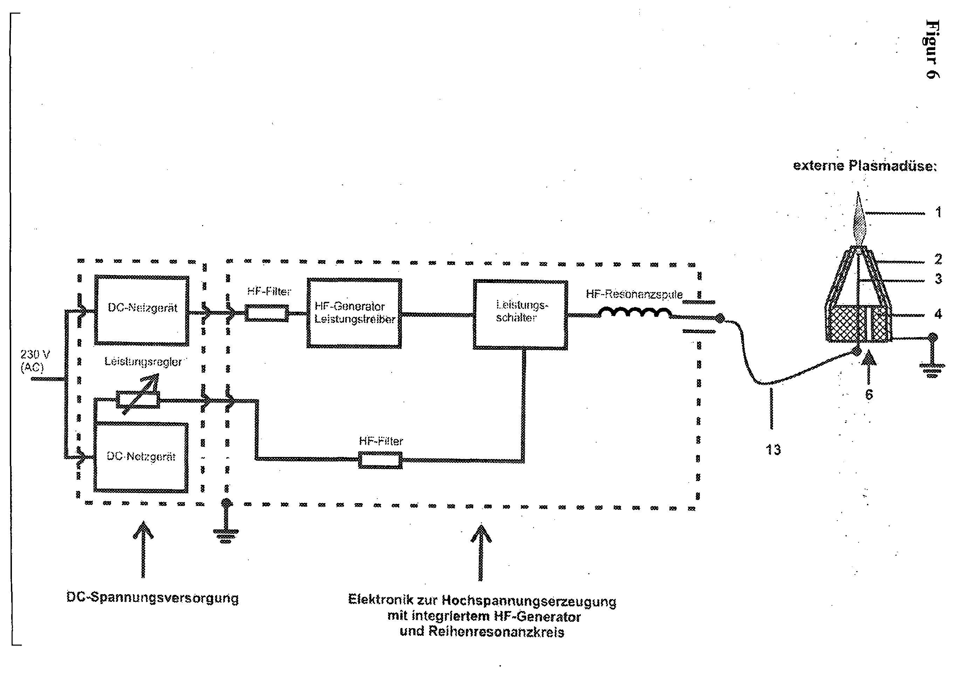

Die Alternative zur Integration von Plasmadüse, Hochspannungsspule und HF-Generator besteht darin, dass man, wie in Fig. 6 dargestellt, die Plasmadüse von der Hochspannungsquelle abkoppelt und über ein geeignetes, vorzugsweise kurzes, Hochspannungskabel sowie über einen entsprechenden Prozessgas-Schlauch extern speist. Diese Anordnung ermöglicht es, die Plasmadüse weiter zu miniaturisieren und damit auch an schwer zugänglichen Stellen eine lokale Plasmabehandlung vornehmen zu können. In diesem Fall werden zusätzliche Maßnahmen zur Vermeidung einer erhöhten elektromagnetischen Störabstrahlung ergriffen.

Diese Anordnung bietet darüber hinaus die Möglichkeit, die Plasmadüse durch eine andere, an das zu behandelnde Objekt angepasste, externe Plasmaquelle zu ersetzen.

Die Erzeugung der für eine Zündung des Plasmas notwendigen Hochfrequenzspannung erfolgt in den, in der Patentschrift des Anmelders "DE 10 2006 019 664 A1" beschriebenen, Plasma-Jet-Anordnungen über eine Luftspule. Zur Abschirmung der Hochfrequenz (Einhaltung der EMV) sowie zur Halterung der Anordnung besteht die Umhüllende in der Regel aus einem metallischen Gehäuse. Das in der Luftspule erzeugte elektromagnetische Feld erzeugt in dem metallischen Gehäuse Wirbelströme, die zum einen zu einer unerwünschten induktiven Erwärmung des Metalls führen und zum anderen als Verlustleistung vom HF-Generator aufzubringen sind (Effizienzerniedrigung).

Zur Vermeidung dieses Effektes wird in dem erfindungsgemäßen Plasmawerkzeug eine Spule mit einem für die Hochfrequenz geeigneten, geschlossenen Ferritkern verwendet, der ein Heraustreten des von der Spule erzeugten Magnetfeldes verhindert.

Gegenstand der Patentanmeldung ist eine Vorrichtung zur Erzeugung eines kalten, HF-angeregten Plasmas unter Atmosphärendruckbedingungen, umfassend einen Hohlkörper für die Zuführung eines Prozessgases, einen Reihenresonanzkreis zur Erzeugung der benötigten Hochspannung und einen HF-Generator, dadurch gekennzeichnet, dass durch die Miniaturisierung und Integration der dafür erforderlichen elektronischen Baugruppen (HF-Generator mit Leistungstreiber, Leistungsschalter, HF-Resonanzspule und HF-Filter) und einer, gegebenenfalls auch als Wechseldüse mit verschiedenen Elektrodenanordnungen gestalteten, Plasmadüse in einem handlichen, metallischen Gehäuse die Einhaltung der Richtlinien der elektromagnetischen Verträglichkeit (EMV) gewährleistet werden kann sowie ein mobiler Einsatz des Gerätes ermöglicht wird.

Gemäß einer bevorzugten Ausführungsform der Erfindung umfasst die Vorrichtung eine elektrisch leitende Kappe (12) die im vorderen Bereich einen Schlitz oder ein Loch (11) aufweist. Der Schlitz oder das Loch sollte höchstens 0,7 mm breit, der Schlitz kann vorzugsweise 8 mm lang sein. Diese Kappe ist elektrisch mit dem Gehäuse (7) verbunden und damit auch geerdet. Das Plasma endet im Bereich des Schlitzes und kann, bedingt durch die Geometrie des Schlitzes, nicht heraustreten, nach dem Prinzip eines Faradayschen Käfigs. Diese Kappe wirkt wie eine dritte Elektrode und leitet den elektrischen Strom zur Erde ab. Damit ist eine Feldfreiheit in unmittelbarer Umgebung des Schlitzes gegeben.

Die zur Behandlung notwendigen Spezies und die Strahlung stehen jedoch, wenn auch etwas gemindert, hinter dem Spalt zur Verfügung. Die antimikrobielle Wirkung konnte nachgewiesen werden.

Gleichzeitig wird durch diese Kappe die thermische Energie aufgenommen und die Temperaturen sind so gering, dass eine permanente Behandlung auf der Haut, auch in sensiblen Bereichen, erfolgen kann.

Der entscheidende Vorteil ist, dass eine solche annehmbare Kappe eine Option für den Anwender darstellt. Benötigt er ein "kräftigeres" Plasma, dann arbeitet er ohne Kappe. Die Kappe wird - je nach Anwendungsgebiet - einfach aufgeschoben oder abgezogen.

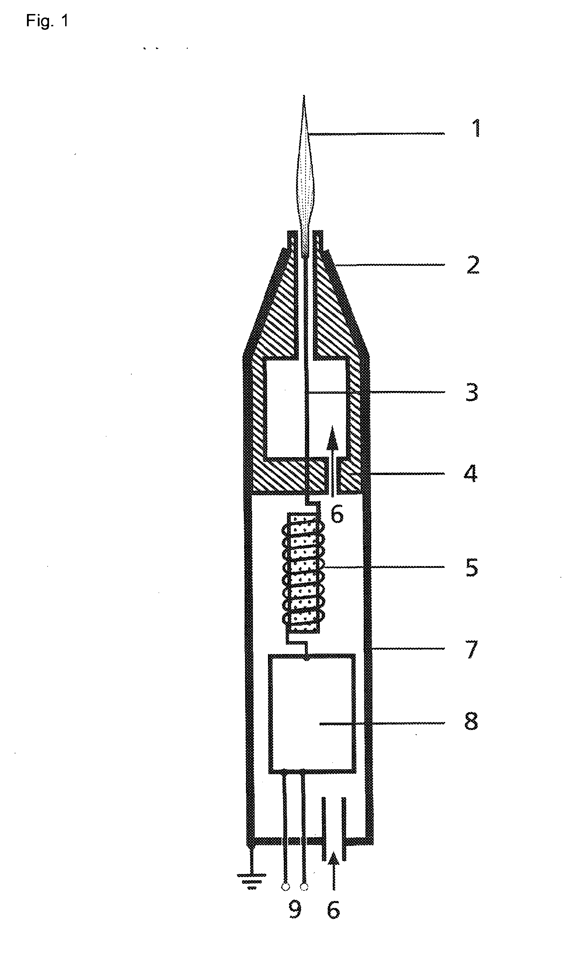

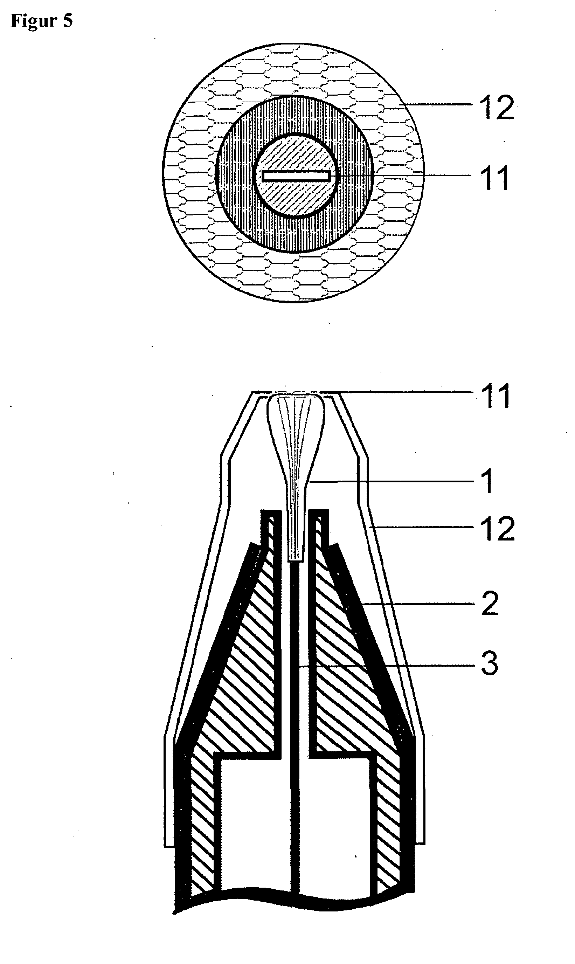

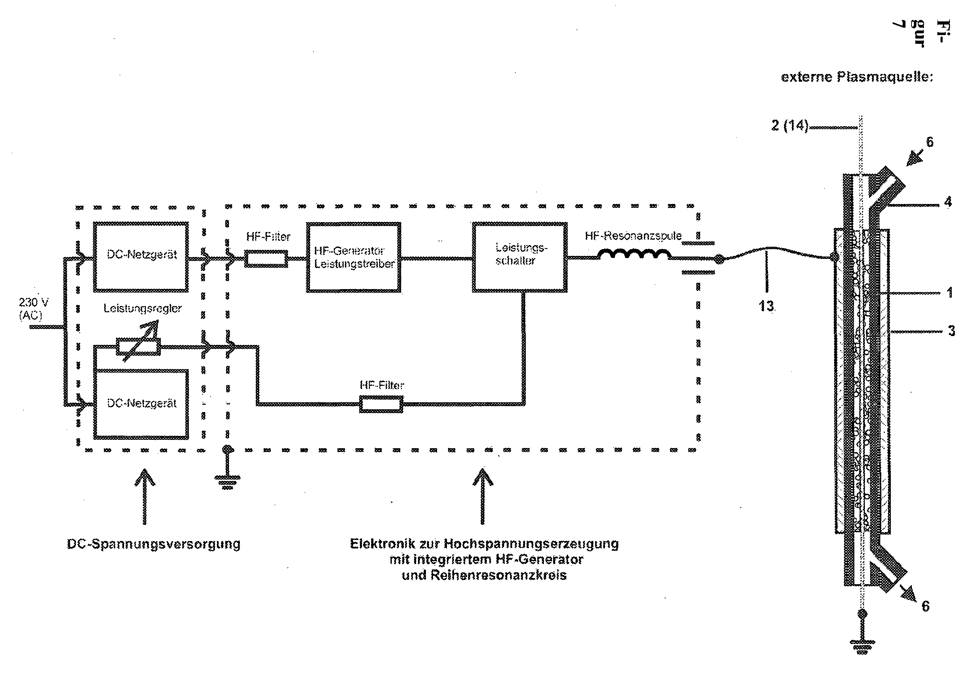

Die Erfindung soll nachfolgend anhand der Figuren 1 bis 7 näher erläutert werden, ohne die Erfindung auf diese Beispiele zu beschränken.

### Ausführungsbeispiele:

Mit den nachfolgend in Fig. 1 bis Fig. 7 dargestellten Zeichnungen wird die Erfindung detailliert erläutert. Für die Kennzeichnung der einzelnen Elemente des Aufbaus der Vorrichtungen werden folgende Bezugszeichen verwendet:

### Bezugszeichenliste:

| | |
|---|---|
| 1 Plasmas | 8 HF-Generator / Leiterplatte |
| 2 Elektrode (geerdetes Gehäuse) | 9 dc-Spannungsanschluss |
| 3 Hochspannungs-Elektrode | 10 Steckkontakt |
| 4 Isolierkörper / Gasdüse | 11 Schlitz |
| 5 HF-Resonanzspule | 12 Kappe |
| 6 Prozessgas | 13 Hochspannungsleitung |
| 7 Gehäuse (Metall) | 14 zu behandelndes, leitfähiges Material (z.B. Draht: mit oder ohne Isolation) |

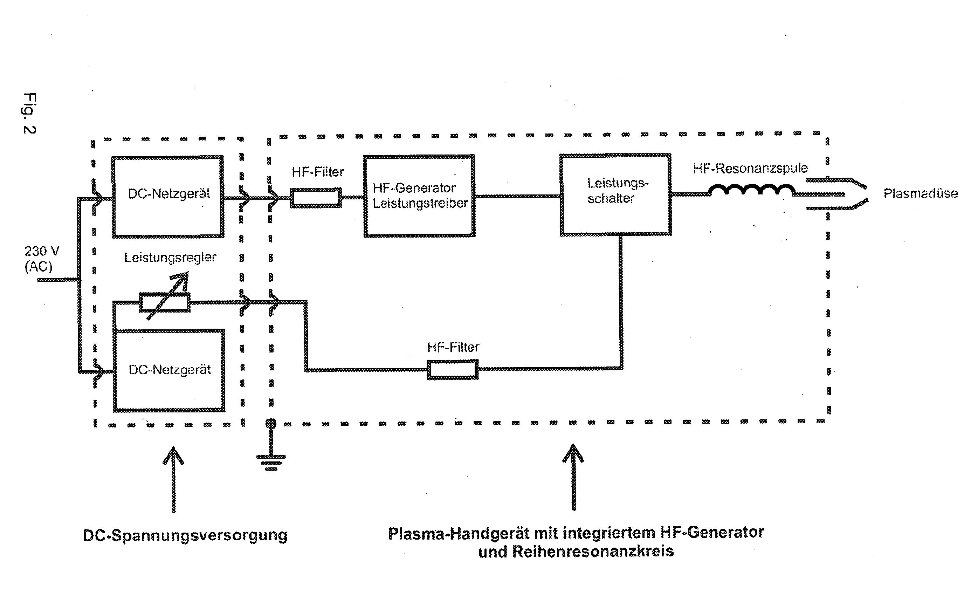

Fig.1 zeigt beispielhaft den prinzipiellen Aufbau der erfindungsgemäßen Vorrichtung eines Plasma-Handgerätes mit integriertem HF-Generator und Reihenresonanzkreis. In einem Metallgehäuse (7), das im Bereich des austretenden Plasmas (1) als geerdete Elektrode (2) wirkt, sind eine Leiterplatte (8) mit EMV-gerechtem Platinenlayout, eine HF-Resonanzspule (5) mit einem, für die Hochfrequenz geeigneten, geschlossenen Ferritkern, ein als Gasdüse fungierender Isolierkörper (4) sowie eine in dem Isolierkörper (4) gehalterte Hochspannungs-Elektrode (3) in der Weise angeordnet, dass sie vom Prozessgas (6) um- bzw. durchströmt werden. Damit wird erreicht, dass das strömende Prozessgas (6) eine Kühlung der Elektronik (8) und der Spule (5) bewirkt. Die Leiterplatte (8) ist dabei im Interesse einer Miniaturisierung sowie eines EMV-gerechten Layouts vorzugsweise mit SMD-Bauteilen bestückt. Das Blockschaltbild der elektronischen Schaltung ist in Fig. 2 dargestellt. Die mit der Leiterplatte (8) realisierte elektronische Schaltung besteht im Wesentlichen aus einem HF-Generator mit einem Leistungstreiber zur Erzeugung einer geeigneten HF-Spannung mit einer Frequenz von circa 1 MHz und einem Leistungsschalter. Zusätzlich werden zwei HF-Filter verwendet, um elektromagnetische Störabstrahlungen nach außen über die Zuleitungen zur außerhalb des Plasma-Handgerätes angeordneten DC-Spannungsversorgung zu vermeiden.

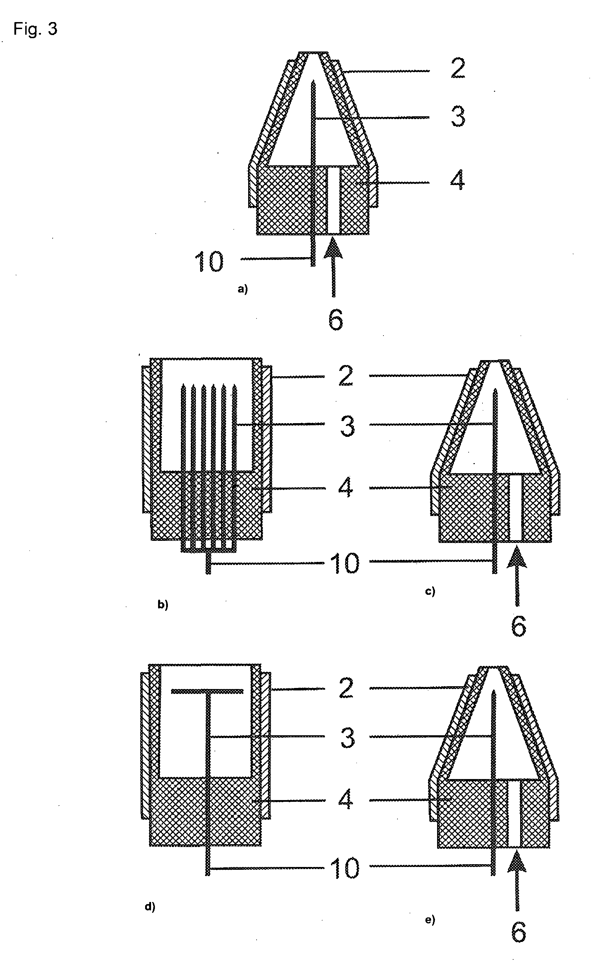

Das in Fig.1 dargestellte Ausführungsbeispiel der erfindungsgemäßen Vorrichtung ist in erster Linie zur Erzeugung eines einzelnen Plasmastrahls mit relativ geringem Durchmesser vorgesehen und ist in dieser Ausführungsform mit einer aus Isoliermaterial aufgebauten Gasdüse (4) vor allem für den Betrieb mit Edelgasen als Prozessgas geeignet. Wie in Fig. 3 dargestellt, kann dabei die mit isolierter geerdeter Elektrode arbeitende Plasmadüse mit verschiedenen Hochspannungs-Elektroden ausgerüstet werden. Neben dem originalen Aufbau mit einer einzelnen nadelförmigen Elektrode (Fig. 3a) ist es zur Verbreiterung des wirksamen Plasmas möglich, entweder eine Anordnung mit mehreren nadelförmigen Elektroden (Fig. 3b: Frontansicht, Fig. 3c: Seitenansicht) oder eine messerförmige Elektrode (Fig. 3d: Frontansicht, Fig. 3e: Seitenansicht) zu verwenden.

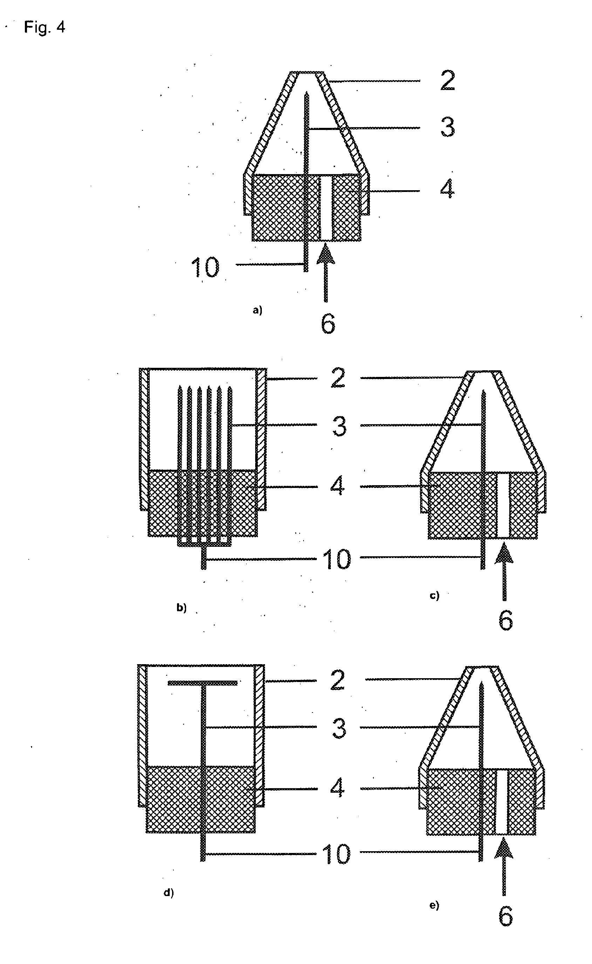

Im Fall des Betriebes mit Molekülgasen (z.B. Luft bzw. N₂) als Prozessgas werden Plasmadüsen verwendet, bei denen die geerdete Elektrode nicht gegenüber dem Gasraum isoliert ist. Beispiele für Ausführungsformen dieser Art von Plasmadüsen sind in Fig. 4 dargestellt (Fig. 4 a: Düse mit einer einzelnen nadelförmigen Elektrode, Fig. 4 b: Frontansicht einer Düse mit mehreren nadelförmigen Elektroden, Fig. 4 c: Seitenansicht einer Düse mit mehreren nadelförmigen Elektroden, Fig. 4 d: Frontansicht einer Düse mit einer messerförmigen Elektrode, Fig. 4 e: Seitenansicht einer Düse mit einer messerförmigen Elektrode). Die verschiedenen Düsen sind dabei derart gestaltet, dass sie auswechselbar sind und die Hochspannungs-Elektroden jeweils über einen Steckkontakt (10) mit der HF-Resonanzspule (5) verbunden werden.

In Fig. 5 ist nochmals der Kopf aus Fig.1 dargestellt. Die besondere Ausführungsform gegenüber dem Kopf aus Fig.1 besteht aus einer elektrisch leitenden Kappe (12) die im vorderen Bereich einen Schlitz oder ein Loch (11) aufweist. Der Schlitz oder das Loch ist höchstens 0,7 mm breit, der Schlitz kann vorzugsweise 8 mm lang sein.

In Fig. 6 ist dargestellt, dass die Plasmadüse von der Hochspannungsquelle abgekoppelt ist und über ein geeignetes, vorzugsweise kurzes Hochspannungskabel sowie über einen entsprechenden Prozessgas-Schlauch extern gespeist wird.

Fig. 7 zeigt als Beispiel eine Anordnung mit einer, speziell für die Plasmabehandlung von isolierten oder nicht isolierten Drähten geeigneten, Plasmaquelle.

## Patentansprüche

1. Vorrichtung für die Erzeugung eines kalten Plasmastrahls, umfassend ein im Bereich des austretenden Plasmas (1) als geerdete Elektrode (2) fungierendes Metallgehäuse (7), in dem ein HF-Generator (8), eine HF-Resonanzspule (5) mit einem, für die Hochfrequenz geeigneten geschlossenen Ferritkern, ein als Gasdüse fungierender Isolierkörper (4) sowie eine in dem Isolierkörper (4) gehalterte Hochspannungs-Elektrode (3) in der Weise angeordnet, dass sie vom Prozessgas (6) um- bzw. durchströmt werden.

2. Vorrichtung nach Anspruch 1, **dadurch gekennzeichnet, dass** sie eine elektrisch leitende abnehmbare Kappe (12) umfasst, die im vorderen Bereich einen Schlitz oder ein Loch (11) aufweist.

3. Vorrichtung nach Anspruch 2, **dadurch gekennzeichnet, dass** der Schlitz oder das Loch höchstens 0,7 mm breit ist.

4. Vorrichtung nach Anspruch 2 oder 3, **dadurch gekennzeichnet, dass** die Kappe elektrisch mit dem Gehäuse (7) verbunden ist.

5. Vorrichtung nach einem der Ansprüche 1 bis 4, **dadurch gekennzeichnet, dass** der HF-Generator (8) in eine Leiterplatte (8) mit EMV-gerechtem Platinenlayout integriert ist.

6. Vorrichtung nach Anspruch 5, **dadurch gekennzeichnet, dass** die Leiterplatte (8) mit SMD-Bauteilen oder anderen Miniaturlösungen wie MEMS (Micro-Electro-Mechanical Systems) bestückt ist.

7. Vorrichtung nach Anspruch 5 oder 6, **dadurch gekennzeichnet, dass** die Leiterplatte (8) einen Leistungstreiber zur Erzeugung einer geeigneten HF-Spannung mit einer Frequenz von circa 1 MHz und einen Leistungsschalter umfasst.

8. Vorrichtung nach einem der Ansprüche 1 bis 7, **dadurch gekennzeichnet, dass** sie HF-Filter besitzt, um elektromagnetische Störabstrahlungen nach außen über die Zuleitungen zur außerhalb des Plasma-Handgerätes angeordneten DC-Spannungsversorgung (9) zu vermeiden.

9. Vorrichtung nach einem der Ansprüche 1 bis 8, **dadurch gekennzeichnet, dass** als Prozessgas Edelgase, Luft, Sauerstoff oder Stickstoff oder beliebige Gemische aus Kombinationen der genannten Gase dienen.

10. Vorrichtung nach einem der Ansprüche 1 bis 9, **dadurch gekennzeichnet, dass** die Hochspannungs-Elektrode (3) eine einzelne nadelförmigen Elektrode oder eine messerförmige Elektrode ist oder mehrere nadelförmigen Elektroden als Hochspannungs-Elektrode (3) dienen.

11. Vorrichtung nach einem der Ansprüche 1 bis 10, **dadurch gekennzeichnet, dass** die Gasdüsen auswechselbar sind und die Hochspannungs-Elektroden (3) jeweils über einen Steckkontakt (10) mit der HF-Resonanzspule (5) verbunden sind.

12. Vorrichtung nach einem der Ansprüche 1 bis 11, **dadurch gekennzeichnet, dass** im Fall des Betriebes mit Molekülgasen (z.B. Luft bzw. Stickstoff) als Prozessgas die geerdete Elektrode (2) nicht gegenüber dem Gasraum isoliert ist.

13. Vorrichtung nach einem der Ansprüche 1 bis 12, **dadurch gekennzeichnet, dass** es sich um ein Handgerät mit zusätzlichem Batteriebetrieb handelt.

14. Vorrichtung nach einem der Ansprüche 1 bis 12, **dadurch gekennzeichnet, dass** die Gasdüse von der Hochspannungsquelle abgekoppelt ist und über ein Hochspannungskabel sowie über einen Prozessgas-Schlauch extern gespeist wird.

## Claims

1. Apparatus for the production of a cold plasma jet, comprising a metal housing (7) that functions as a grounded electrode (2), in the region of the exiting plasma (1), in which housing an HF generator (8), an HF resonance coil (5) having a ferrite core suitable for the high frequency, an insulation body (4) that functions as a gas nozzle, as well as a high-voltage electrode (3) mounted in the insulation body (4) are disposed in such a manner that the process gas (6) flows around and through them.

2. Apparatus according to claim 1, **characterized in that** it comprises an electrically conductive removable cap (12), which has a slit or a hole (11) in the front region.

3. Apparatus according to claim 2, **characterized in that** the slit or the hole has a width of at most 0.7 mm.

4. Apparatus according to claim 2 or 3, **characterized in that** the cap is electrically connected with the housing (7).

5. Apparatus according to one of claims 1 to 4, **characterized in that** the HF generator (8) is integrated into a circuit board (8) having an EMT-appropriate board layout.

6. Apparatus according to claim 5, **characterized in that** the circuit board (8) is equipped with SMD components or other miniature solutions such as MEMS (micro-electro-mechanical systems).

7. Apparatus according to claim 5 or 6, **characterized in that** the circuit board (8) comprises a power driver for the production of a suitable HF voltage having a frequency of approximately 1 MHz and a power switch.

8. Apparatus according to one of claims 1 to 7, **characterized in that** it possesses HF filters to avoid electromagnetic interference radiation toward the outside, by way of the feed lines to the DC voltage supply (9) disposed outside the hand-held plasma device.

9. Apparatus according to one of claims 1 to 8, **characterized in that** noble gases, air, oxygen or nitrogen or any desired mixtures of combinations of the said gases serve as the process gas.

10. Apparatus according to one of claims 1 to 9, **characterized in that** the high-voltage electrode (3) is a single, needle-shaped electrode or a blade-shaped electrode, or that multiple needle-shaped electrodes serve as the high-voltage electrode (3).

11. Apparatus according to one of claims 1 to 10, **characterized in that** the gas nozzles are interchangeable and that the high-voltage electrodes (3) are connected with the HF resonance coil (5) by way of a plug-in contact (10), in each instance.

12. Apparatus according to one of claims 1 to 11, **characterized in that** in the event of operation with molecular gases (e.g. air or nitrogen) as the process gas, the grounded electrode (2) is not insulated relative to the gas space.

13. Apparatus according to one of claims 1 to 12, **characterized in that** it is a hand-held device with additional battery operation.

14. Apparatus according to one of claims 1 to 12, **characterized in that** the gas nozzle is uncoupled from the high-voltage source and is supplied externally by way of a high-voltage cable as well as by way of a process gas hose.

## Revendications

1. Dispositif pour la génération d'un jet de plasma froid comportant un boîtier métallique (7) faisant fonction d'électrode mise à la terre (2) dans la région du plasma sortant (1), dans lequel sont agencés un générateur HF (8), une bobine de résonance HF (5) avec un noyau de ferrite fermé approprié pour la haute fréquence, un corps isolant (4) faisant fonction de buse à gaz ainsi qu'une électrode à haute tension (3) maintenue dans le corps isolant (4), de sorte qu'ils sont entouré et/ou parcouru par le gaz de processus (6).

2. Dispositif selon la revendication 1, **caractérisé en ce qu'**il comprend un capuchon amovible électriquement conducteur (12) comportant une fente ou un trou (11) dans la partie avant.

3. Dispositif selon la revendication 2, **caractérisé en ce que** la fente ou le trou a une largeur de 0.7 mm au maximum.

4. Dispositif selon la revendication 2 ou 3, **caractérisé en ce que** le capuchon est électriquement relié au boîtier (7).

5. Dispositif selon l'une quelconque des revendications 1 à 4, **caractérisé en ce que** le générateur HF (8) est intégré dans une carte imprimée (8) avec une conception de carte de circuit selon les prescriptions CEM.

6. Dispositif selon la revendication 5, **caractérisé en ce que** la carte imprimée (8) est équipée des composants SMD ou des autres solutions miniaturisées comme MEMS (Micro-Electro-Mechanical Systems).

7. Dispositif selon la revendication 5 ou 6, **caractérisé en ce que** la carte imprimée (8) comprend un pilote de performance pour la génération d'une tension HF appropriée avec une fréquence de 1 MHz environ et un disjoncteur.

8. Dispositif selon l'une quelconque des revendications 1 à 7, **caractérisé en ce qu'**il comprend des filtres HF pour éviter des émissions électromagnétiques d'interférence vers l'extérieur à travers les conduites d'amenée à l'alimentation DC (9) agencée à l'extérieur de l'appareil portatif de plasma.

9. Dispositif selon l'une quelconque des revendications 1 à 8, **caractérisé en ce que** des gaz rares, d'air, d'oxygène ou d'azote ou des mélanges quelconques de combinaisons des gaz mentionnés servent en tant que gaz de processus.

10. Dispositif selon l'une quelconque des revendications 1 à 9, **caractérisé en ce que** l'électrode à haute tension (3) est une électrode individuelle en forme d'aiguille ou une électrode en forme de couteau ou plusieurs électrodes en forme d'aiguille servent en tant qu'électrode à haute tension (3).

11. Dispositif selon l'une quelconque des revendications 1 à 10, **caractérisé en ce que** les buses à gaz sont interchangeable et les électrodes à haute tension (3) sont reliées chacune avec la bobine de résonance HF (5) à travers un contact mâle (10).

12. Dispositif selon l'une quelconque des revendications 1 à 11, **caractérisé en ce que** dans le cas de fonctionnement avec des gaz à molécules (par exemple d'air et/ou d'azote) en tant que gaz de processus, l'électrode mise à la terre (2) n'est pas isolée vis-à-vis la chambre à gaz.

13. Dispositif selon l'une quelconque des revendications 1 à 12, **caractérisé en ce qu'**il s'agit d'un appareil portatif avec batterie de sauvegarde.

14. Dispositif selon l'une quelconque des revendications 1 à 12, **caractérisé en ce que** la buse à gaz est découplée de la source de haute tension et est alimentée en externe à travers un câble électrique haute tension ainsi qu'à travers un tuyau de gaz de processus.
